# EUROPEAN PATENT APPLICATION

(11) **EP 4 549 942 A1**
(43) Date of publication of application: **07.05.2025**
(21) Application number: 23831123.7
(22) Date of filing: 15.06.2023
(51) Int. Cl.: G01N 33/574, C12Q 1/68

(54) **METHOD FOR CHECKING POSSIBILITY OF LIVER CANCER ONSET**

(30) Priority: 01.07.2022 JP 2022106996
(71) Applicant: OSAKA UNIVERSITY, Suita-shi Osaka 565-0871 (JP)
(72) Inventor: HIKITA, Hayato, Suita-shi, Osaka 565-0871 (JP); SAKANE, Sadatsugu, Suita-shi, Osaka 565-0871 (JP); TAKEHARA, Tetsuo, Suita-shi, Osaka 565-0871 (JP); FUKUMOTO, Kenji, Suita-shi, Osaka 565-0871 (JP)
(74) Representative: Marks & Clerk LLP
(86) International application number: PCT/JP2023/022224
(87) International publication number: WO 2024/004675

(57) **Abstract**

The present invention provides a method for testing a possibility of liver cancer development. A method for testing a possibility of liver cancer development includes step (1) of detecting at least one target molecule selected from the group consisting of Fibulin-3, GPNMB, Fibulin-3 mRNA, and GPNMB mRNA in a biological sample collected from a subject.

## Description

### Technical Field

The present invention relates to a method for testing a possibility of liver cancer development, for example.

### Background Art

Liver cancer is a malignant tumor that affects approximately 40,000 people and kills 25,000 people each year in Japan. Most patients affected with liver cancer have some chronic liver disease, and treatment for the chronic liver disease is important for improving the prognosis of liver cancer patients. In patients with chronic hepatitis C, novel therapeutic drugs have enabled the elimination of virus in many cases; however, hepatocarcinogenesis develops even after the elimination of the virus in some cases. Also with respect to chronic hepatitis B, there are several therapeutic drugs; however, cases have been found in which patients develop liver cancer even when pharmaceutical drugs are orally administrated. In cases of non-alcoholic fatty liver disease and alcoholic hepatic disorder, there is still no treatment other than weight loss and abstinence from alcohol, and cases have been found in which patients develop liver cancer even after weight loss and abstinence from alcohol. As described above, current chronic liver disease treatment alone reduces liver cancer occurrence but cannot completely prevent liver cancer development. Under the present circumstances, current medical care provides patients with chronic liver disease with follow-up using regular blood tests and imaging tests for early detection of liver cancer. Under such circumstances, a marker that can predict future hepatocarcinogenesis development with a high percentage is very useful because the marker enables early detection of liver cancer through strict follow-up in high-risk groups and also reduces the burden on patients and the medical economy by reducing test frequency in low-risk groups.

α-fetoprotein or The FIB4-index, which is calculated from AST, ALT, platelets, and age, has been reported as a predictive marker for carcinogenesis at present; however, its diagnostic ability is not sufficient (NPL 1).

### Citation List

### Non-patent Literature

NPL 1: Aliment Pharmacol Ther 2021 Nov; 54(10):1340-1349. doi: 10.1111/apt.16632.

### Summary of Invention

### Technical Problem

An object of the present invention is to provide a method for testing a possibility of liver cancer development.

### Solution to Problem

The present inventors have conducted extensive research in view of the object described above, and thus have found that the object can be achieved by a method for testing a possibility of liver cancer development including step (1) of detecting at least one target molecule selected from the group consisting of Fibulin-3, GPNMB, Fibulin-3 mRNA, and GPNMB mRNA in a biological sample collected from a subject. The present inventors have further conducted research based on this finding, and thus have accomplished the present invention. More specifically, the present invention encompasses the following aspects.

### Item 1.

A method for testing a possibility of liver cancer development, including step (1) of detecting at least one target molecule selected from the group consisting of Fibulin-3, GPNMB, Fibulin-3 mRNA, and GPNMB mRNA in a biological sample collected from a subject.

### Item 2.

The method according to Item 1, in which step (1) includes:
step (1a) of bringing a binding molecule to the target molecule and the biological sample into contact with each other; and
step (1b) of measuring the amount or the concentration of the target molecule bonded to the binding molecule.

### Item 3.

The method according to Item 1, further including step (2) of determining the possibility of liver cancer development of the subject based on the amount or the concentration of the target molecule detected in step (1).

### Item 4.

The method according to Item 3, in which step (2) includes:
step (2a) of determining that, when the amount or the concentration of the target molecule detected in step (1) is equal to or greater than a cutoff value, the possibility of liver cancer development of the subject is high; and/or
step (2b) of determining that, when the amount or the concentration of the target molecule detected in step (1) is equal to or smaller than the cutoff value, the possibility of liver cancer development of the subject is low.

### Item 5.

The method according to any one of Items 1 to 4, in which the subject is a subject having chronic liver disease.

### Item 6.

The method according to any one of Items 1 to 4, in which the target molecule is at least one member selected from the group consisting of Fibulin-3 and GPNMB.

### Item 7.

The method according to any one of Items 1 to 4, in which the biological sample is a body fluid or a sample derived from a body fluid.

### Item 8.

The method according to Item 7, in which the body fluid is at least one member selected from the group consisting of whole blood, serum, and plasma.

### Item 9.

The method according to any one of Items 1 to 4, in which the subject is a human.

### Item 10.

A test drug for a possibility of liver cancer development, containing a binding molecule to at least one target molecule selected from the group consisting of Fibulin-3, GPNMB, Fibulin-3 mRNA, and GPNMB mRNA.

### Item 11.

Use of a binding molecule to at least one target molecule selected from the group consisting of Fibulin-3, GPNMB, Fibulin-3 mRNA, and GPNMB mRNA for producing a test drug for a possibility of liver cancer development.

### Item. 12.

Use of a binding molecule to at least one target molecule selected from the group consisting of Fibulin-3, GPNMB, Fibulin-3 mRNA, and GPNMB mRNA as a test drug for a possibility of liver cancer development.

### Item 13.

A binding molecule to at least one target molecule selected from the group consisting of Fibulin-3, GPNMB, Fibulin-3 mRNA, and GPNMB mRNA for use in testing a possibility of liver cancer development.

### Advantageous Effects of Invention

The present invention can provide a method for testing a possibility of liver cancer development.

### Brief Description of Drawings

Fig. 1 illustrates the cumulative hepatocarcinogenesis occurrence of all non-alcoholic fatty liver disease patients in Test Example 1.
Fig. 2 illustrates the serum Fibulin-3 values in each patient group in Test Example 1, in which F0 to F4 indicate the fibrosis stages, non-HCC (hepatocellular carcinoma) indicates a non-carcinogenesis group, and HCC indicates a carcinogenesis group (**** p < 0.0001, ** p < 0.01).
Fig. 3 illustrates the cumulative hepatocarcinogenesis occurrence of each of two groups classified according to the serum Fibulin-3 value (145 patients with less than 9.0 µg/mL and 25 patients with 9.0 µg/mL or more) in Test Example 1.
Fig. 4 illustrates the cumulative hepatocarcinogenesis occurrence after achieving sustained virologic response (SVR) in chronic hepatitis C in Test Example 2.
Fig. 5 illustrates the distribution of the serum Fibulin-3 values before treatment for chronic hepatitis C in Test Example 2.
Fig. 6 illustrates the serum Fibulin-3 values in each patient group in Test Example 2, in which F0 to F4 indicate the fibrosis stages, non-HCC indicates a non-carcinogenesis group, and HCC indicates a carcinogenesis group (**** p < 0.0001, *** p < 0.001).
Fig. 7 illustrates the cumulative hepatocarcinogenesis occurrence of each of two groups classified according to the serum Fibulin-3 value (348 patients with less than 15.0 µg/mL and 122 patients with 15.0 µg/mL or more) in Test Example 2.
Fig. 8 illustrates the cumulative hepatocarcinogenesis occurrence of each of two patient groups classified according to the serum Fibulin-3 value (348 patients with less than 15.0 µg/mL and 122 patients with 15.0 µg/mL or more) when the patient groups in Test Example 2 were limited to a patient group with an FIB4 index of less than 3.25, or F1 and F2 patient groups.
Fig. 9 illustrates the serum GPNMB values of each patient group in Test Example 3.
Fig. 10 illustrates the cumulative hepatocarcinogenesis occurrence of each of two groups classified according to the serum GPNMB value (15.4 µg/mL or less, and more than 15.4 µg/mL) in Test Example 3.
Fig. 11 illustrates the serum GPNMB values in a non-carcinogenesis group and a carcinogenesis group in Test Example 4.
Fig. 12 illustrates the cumulative hepatocarcinogenesis occurrence of each of two groups classified according to the serum GPNMB value (15.4 µg/mL or less, and more than 15.4 µg/mL) in Test Example 4.
Fig. 13 illustrates the results of calculating the area under the ROC curve (AUROC) based on the time-dependent ROC curve for both the serum Fibulin-3 value and the FIB-4 index in Test Example 5.
Fig. 14 illustrates the cumulative hepatocarcinogenesis occurrence of each of two groups classified according to the Fibulin-3 value (6.7 µg/mL or more, and less than 6.7 µg/mL) in the cases of a total of 476 patients, 120 patients with fibrosis extension, and 120 patients with a FIB-4 index of 2.67 or more in Test Example 5.

### Description of Embodiments

In this specification, the expressions "contain" and "include" include the concepts of containing, including, substantially consisting of, and consisting only of.

### 1. Method for testing possibility of liver cancer development

A method for testing a possibility of liver cancer development includes step (1) of detecting at least one target molecule selected from the group consisting of Fibulin-3, GPNMB, Fibulin-3 mRNA, and GPNMB mRNA (in this specification, sometimes referred to as a "target molecule of the present invention") in a biological sample collected from a subject (in this specification, sometimes referred to as "test method of the present invention"). The method is described below.

### 1-1. Step (1)

Liver cancer to be tested is not particularly restricted and encompasses hepatocellular carcinoma, cholangiocarcinoma, and the like. As liver cancer, hepatocellular carcinoma is preferable. Liver cancer encompasses liver cancers of all classes, grades, and stages in various classification criteria for stages, conditions, and the like.

The subject may be any mammal, and is preferably a mammal with chronic liver disease. Examples of the mammals include rodents, such as mice, rats, hamsters, and guinea pigs; laboratory animals, such as rabbits; pets, such as dogs and cats; livestock, such as cows, pigs, goats, horses, and sheep; primates, such as monkeys, orangutans, and chimpanzees; humans; and the like, with humans being particularly preferable.

The chronic liver disease includes, but is not limited to, viral hepatitis and fatty liver disease. The viral hepatitis includes, but is not limited to, hepatitis C and hepatitis B. The fatty liver disease includes, but is not limited to, non-alcoholic fatty liver disease (NAFLD) and secondary fatty liver. NAFLD includes non-alcoholic fatty liver (NAFL) and non-alcoholic steatohepatitis (NASH). The subject in the present invention includes a patient with chronic liver disease who is required to be assessed for a risk of liver cancer development. The subject in the present invention includes, but is not limited to, a subject after achieving sustained virologic response (SVR) in hepatitis C, a subject under nucleoside analogue (NUC) administration in hepatitis B, and a subject with NAFLD. In the present invention, the sustained virological response (SVR) in hepatitis C virus (HCV), treatment for achieving SVR, and a test for checking the development or non-development of liver cancer after achieving SVR follow definitions by authoritative experts on hepatitis and/or liver cancer, including, but not limited to, the definitions in the following literature: Hepatitis C Treatment Guideline (edited by the Hepatitis Treatment Guideline Preparation Committee of the Japan Society of Hepatology, 8th edition, published in July 2020) (https://www.jsh.or.jp/lib/files/medical/guidelines/jsh-guidlines /C_v8_20201005.pdf)) and its English version (Hepatology Research 2020; 50: 791-816)); Clinical Practice Guidelines for Hepatocellular Carcinoma (edited by the Japan Society of Hepatology, 2017 edition, published in October 2017) (https://www.jsh.or.jp/medical/guidelines/jsh_guidlines/medical/e -xamination_jp_2017.html)) and its English version (https://www.jsh.or.jp/English/examination_en/guidelines_hepatoce -llular_carcinoma_2017.html); Ghany MG and Morgan TR, Hepatitis C Guidance 2019 Update: American Association for the Study of Liver Diseases-Infectious Diseases Society of America Recommendations for Testing, Managing, and Treating Hepatitis C Virus Infection. Hepatology 2020; 71:686-721; and Clinical Practice Guidelines Panel (EASL recommendations on treatment of hepatitis C: Final update of the series. J Hepatol 2020; 73:1170-1218).

In the present invention, the treatment by the administration of the NUC in hepatitis B follows the definitions provided by authoritative hepatitis experts including, but not limited to, the definitions provided by Guidelines for the Management of Hepatitis B edited by the Japan Society of Hepatology (Version 3.4), May 2021 (https://www.jsh.or.jp/lib/files/medical/guidelines/jsh-guidlines /B_v3.4.pdf) and its English version (Hepatology Research, 2020; 50: 892-923).

In the present invention, fatty liver disease, non-alcoholic fatty liver disease (NAFLD), non-alcoholic fatty liver (NAFL), and non-alcoholic steatohepatitis (NASH) follow the definitions provided by authoritative hepatitis experts including, but not limited to, the definitions by Clinical Practice Guidelines for NAFLD/NASH 2020 (edited by the Japanese Society of Gastroenterology and the Japan Society of Hepatology, revised second edition, published in November 2020, Nankodo (https://www.jsge.or.jp/guideline/guideline/pdf/nafldnash2020.pdf )) and its English version (Tokushige, K. et al., Hepatology Research, 2021; 51:1013-1025; and Tokushige, K. et al., Journal of Gastroenterology, 2021; 56: 951-963).

In the present invention, a test for checking the development or non-development of liver cancer from chronic viral hepatitis, fatty liver disease, and other liver diseases follows the definitions provided by authoritative hepatitis experts including, but not limited to, the definitions provided by Clinical Practice Guidelines for Hepatocellular Carcinoma (edited by the Japan Society of Hepatology, 2017 edition, listed above).

The biological sample is not particularly restricted insofar as it can contain the target molecule of the present invention. Examples of the biological sample include body fluids, such as whole blood, serum, plasma, follicular fluid, menstrual blood, saliva, cerebrospinal fluid, synovial fluid, urine, tissue fluid, sweat, tears, and saliva, and samples derived from these body fluids. The biological sample can also be a biological tissue, preferably liver tissue, or a sample derived from the tissues. The samples derived from body fluids or tissues are not particularly restricted insofar as the samples are prepared from body fluids or tissues. Examples include samples obtained from body fluids or tissues by concentrating and purifying, for example, proteins or nucleic acids contained in body fluids or tissues and the like. Preferable examples of body fluids include whole blood, serum, plasma, and the like. The biological samples may be used alone or in combination of two or more types.

The biological sample can be collected from the subject by methods known to those skilled in the art. For example, the whole blood can be collected by collecting blood using a syringe or the like. The blood is preferably collected by medical professionals, such as doctors or nurses. The serum is a portion obtained by removing blood cells and specific blood clotting factors from blood and can be obtained as a supernatant after blood has been allowed to clot, for example. The plasma is a portion obtained by removing blood cells from blood and can be obtained as a supernatant when blood is centrifuged under conditions in which the blood does not clot, for example.

Fibulin-3 gene is known, and, in the case of a human, the gene represented by NCBI Gene ID: 2202 is Fibulin-3 gene, for example. Fibulin-3 gene of another species can also be easily identified according to known information and/or based on the identity analysis with the known amino acid sequence and/or base sequence of Fibulin-3 gene. The amino acid sequence of Fibulin-3 can be easily identified according to known information and/or based on the identity analysis with the known amino acid sequence of Fibulin-3. Examples of the amino acid sequence of human Fibulin-3 include the amino acid sequence represented by NCBI Reference No. NP_001034437.1. The base sequence of Fibulin-3 mRNA can be easily identified according to known information and/or based on the identity analysis with the known base sequence of Fibulin-3 mRNA. Examples of the base sequence of human Fibulin-3 mRNA include the base sequence represented by NCBI Reference No. NM_001039348.1.

GPNMB gene is known, and, in the case of a human, the gene represented by NCBI Gene ID: 10457 is GPNMB gene, for example. GPNMB gene of another species can also be easily identified according to known information and/or based on the identity analysis with the known amino acid sequence and/or base sequence of GPNMB gene. The amino acid sequence of GPNMB can be easily identified according to known information and/or based on the identity analysis with the known amino acid sequence of GPNMB. Examples of the amino acid sequence of human GPNMB include the amino acid sequence represented by NCBI Reference No. NP_001005340.2. The base sequence of GPNMB mRNA can be easily identified according to known information and/or based on the identity analysis with the known base sequence of GPNMB mRNA. Examples of the base sequence that produces human GPNMB mRNA include the base sequence represented by NCBI Reference No. NM_001005340.1.

The target molecule of the present invention to be detected in step (1) also encompasses isoforms and those including variations found among individuals.

When the target molecule (Fibulin-3, GPNMB) of the present invention, which is protein, is detected, the method for detecting the target molecule of the present invention (preferably a method for measuring the amount or the concentration of the target molecule of the present invention) is not particularly restricted insofar as the method is capable of detecting the target molecule of the present invention. The method includes immunoassay, for example. A wide variety of immunoassay can be used, including direct immunoassay, indirect immunoassay, homogenous immunoassay, heterogeneous immunoassay, competitive immunoassay, non-competitive immunoassay, and the like. More specifically, examples of the immunoassay include ELISA (e.g., direct ELISA, indirect ELISA, sandwich ELISA, competitive ELISA, and the like), radio immunoassay (RIA), immunoradiometric assay (IRMA), enzyme immunoassay (EIA), sandwich EIA, immunochromatography, Western blotting, immunoprecipitation, slot or dot blot assay, immunohistochemical staining, fluorescence immunoassay, immunoassay using an avidin-biotin or streptavidin-biotin system, immunoassay using surface plasmon resonance (SPR), and the like. The target molecule of the present invention can be detected by the immunoassay, specifically directly or indirectly bringing a labeled antibody into contact with a binding molecule to the target molecule of the present invention bonded to the target molecule of the present invention and quantitatively determining a signal derived from the label of the bonded labeled antibody, for example. The labeled antibody and an antibody mediating the labeled antibody and the binding molecule to the target molecule of the present invention or the target molecule of the present invention are not particularly restricted, and an antibody binding to the antibody constant region, an anti-idiotype antibody, and the like can be used, for example.

The type of the label in a labeled substance (e.g., labeled antibody) used to detect the target molecule of the present invention, which is protein, is not particularly restricted. Examples of the label include fluorescent substances, luminescent substances, dyes, enzymes, gold colloids, radioisotopes, and the like. Among the above, enzyme labels, such as peroxidase and alkaline phosphatase, are preferable from the viewpoint of safety, cost effectiveness, detection sensitivity, and the like.

When the target molecule of the present invention, which is mRNA (Fibulin-3 mRNA, GPNMB mRNA), is detected, the method for detecting the target molecule of the present invention (preferably a method for measuring the amount or the concentration of the target molecule of the present invention) is not particularly restricted insofar as the method is capable of detecting the target molecule of the present invention. As the method, northern blotting, RNase protection assay, reverse transcription polymerase chain reaction (RT-PCR) (Weis JH et al., Trends in Genetics, 1992; 8:263-264); quantitative real-time RT-PCR (Held CA et al., Genome Research, 1996; 6:986-994), and the like, can be used, for example. Quantitative real-time RT-PCR is preferable from the viewpoint of sensitivity and ease of implementation. In the methods above, the binding molecule (e.g., primer, probe, and the like) to the target molecule of the present invention can be used. Primer pairs and probes can be synthesized based on the nucleotide sequence of mRNA of the target molecule of the present invention. The base lengths of the primers and the probes are not particularly limited. The base lengths of the primers each can be set to 10 to 50 nucleotides and preferably set to 15 and 30 nucleotides. The base lengths of the probes can be set in the range of from 10 nucleotides to the full length of the nucleotide sequence complementary to the nucleotide sequence of mRNA of the target molecule of the present invention and preferably can be set to 20 to 150 nucleotides.

As the primer pair and the probe, natural nucleic acids, such as RNA and DNA, can be used, and the natural nucleic acids and chemically modified nucleic acids or pseudo-nucleic acids can be used in combination as required. Examples of the chemically modified nucleic acids and the pseudo-nucleic acids include peptide nucleic acid (PNA), Locked Nucleic Acid (LNA; registered trademark), methyl phosphonate DNA, phosphorothioate DNA, 2'-O-methyl RNA, and the like. The primers and the probes may be labeled or modified using a labeling substance, such as fluorescent substances and/or quencher substances or radioactive isotopes (e.g., ³²P, ³³P, ³⁵S), or modifiers, such as biotin or (strept)avidin or magnetic beads. The labeling substances are not limited, and commercially available substances can be used. For example, as the fluorescent substances, FITC, Texas, Cy3, Cy5, Cy7, Cyanine3, Cyanine5, Cyanine7, FAM, HEX, VIC, fluorescamine and its derivatives, rhodamine and its derivatives, and the like can be used. As the quencher substances, AMRA, DABCYL, BHQ-1, BHQ-2, BHQ-3, or the like can be used. The labeling positions of the labeling substances in the primers and the probes may be determined as appropriate according to the properties of the modifiers and the intended use. In general, the modifications are made to the 5' or 3' end in many cases. One primer molecule and one probe molecule may be labeled with one or more types of labeling substance. The designs of the nucleotide sequences of the primers and the probes and the selection of the labeling substances are known and are disclosed in books on experimental protocols of molecular biology, such as Molecular Cloning: A Laboratory Manual by Sambrook, J and Russell, DW (3rd ed., Cold Spring Harbor Laboratory Press, 2001).

The detection methods may be used alone or in combination of two or more types.

In one aspect, step (1) can preferably include step (1a) of bringing the binding molecule to the target molecule of the present invention and the biological sample into contact with each other, and step (1b) of measuring the amount or the concentration of the target molecule bonded to the binding molecule of the present invention.

The binding molecule to the target molecule of the present invention is not particularly limited insofar as the binding molecule selectively (specifically) recognizes the target molecule of the present invention. Herein, the description "selectively (specifically) recognize" means that the target molecule of the present invention can be specifically detected by Western blotting, ELISA, and the like, for example, but is not limited thereto, and may be such that those skilled in the art can determine that the detected substance is derived from the target molecule of the present invention.

The binding molecule to the target molecule of the present invention encompasses a polyclonal antibody, a monoclonal antibody, a chimeric antibody, a single-chain antibody, or a molecule containing part of the antibodies above having antigen-binding properties, such as Fab fragments or fragments produced by a Fab expression library. The binding molecule to the target molecule of the present invention also includes a binding molecule to the target molecule of the present invention having antigen binding properties to polypeptides containing, at least contiguously, usually 8 amino acids, preferably 15 amino acids, and more preferably 20 amino acids of the amino acid sequence of the target molecule.

Methods for producing the binding molecule to the target molecule of the present invention are already well known, and the binding molecule to the target molecule of the present invention can also be produced according to common methods (Current Protocols in Molecular Biology, Chapter 11.12 to 11.13 (2000)). Specifically, when the binding molecule to the target molecule of the present invention is a polyclonal antibody, the polyclonal antibody can be obtained according to a common method from the serum of an immunized animal, the animal being a nonhuman animal, such as a domestic rabbit, and obtained by being immunized using the target molecule of the present invention expressed in *E. coli* or the like and purified according to a common method or an oligopeptide having a partial amino acid sequence of the target molecule of the present invention synthesized according to a common method. When the binding molecule to the target molecule of the present invention is a monoclonal antibody, the monoclonal antibody can be obtained from hybridoma cells prepared by cell fusion of spleen cells and myeloma cells obtained by immunizing a nonhuman animal, such as a mouse, with the target molecule of the present invention expressed in *E. coli* or the like and purified according to a common method or an oligopeptide having a partial amino acid sequence of the target molecule of the present invention (Current Protocols in Molecular Biology, edited by Ausubel et al., published 1987 by John Wiley and Sons, section 11.4 to 11.11).

The target molecule of the present invention used as an immunogen in the production of the binding molecule to the target molecule of the present invention can be obtained by operations of DNA cloning, construction of each plasmid, transfection into a host, culturing of a transformant, and recovery of proteins from a culture based on known gene sequence information. These operations can be performed according to methods known to those skilled in the art or methods described in the literature (Molecular Cloning, T. Maniatis et al., CSH Laboratory (1983); DNA Cloning, DM Glover, IRL PRESS (1985)), for example.

Specifically, recombinant DNA (expression vector) is produced that allows a gene encoding the target molecule of the present invention to perform expression in a desired host cell, the recombinant DNA is introduced into the host cell for transformation, the transformant is cultured, and the target protein is recovered from the resulting culture, whereby a protein as an immunogen for producing the binding molecule to the target molecule of the present invention can be obtained. A partial peptide of the target molecule of the present invention can also be produced by a common chemical synthesis method (peptide synthesis) according to known gene sequence information.

The binding molecule to the target molecule of the present invention may also be one prepared using an oligopeptide having a partial amino acid sequence of the target molecule of the present invention. Although the oligo(poly)peptide used for producing the binding molecule to the target molecule of the present invention is not required to have functional biological activity, the oligo(poly)peptide desirably has immunogenic properties similar to those of the target molecule of the present invention. Oligo(poly)peptides preferably having the immunogenic properties and containing contiguously at least 8 amino acids, preferably 15 amino acids, and more preferably 20 amino acids in the amino acid sequence of the target molecule of the present invention can be given as examples.

The production of the binding molecule to the target molecule of the present invention for such oligo(poly)peptides can also be performed by enhancing the immunological response using various adjuvants according to the host. Such adjuvants include, but are not limited to, Freund's adjuvant, mineral gels, such as aluminum hydroxide, surfactants, such as lysolecithin, Pluronic polyol, polyanion, peptide, oil emulsion, keyhole limpet hemocyanin, and dinitrophenol, human adjuvants, such as BCG (bacillus Calmette-Guérin) and *Corynebacterium parvum.*

The mode of the contact in step (1a) is not particularly restricted and an appropriate mode can be selected according to the type of the method for detecting the target molecule of the present invention described above (e.g., various kinds of immunoassays and the like). Examples of the contact mode include a contact mode in a state in which only either the protein (antigen) in the biological sample or the binding molecule to the target molecule of the present invention is immobilized on a solid phase, a contact mode in which neither the protein (antigen) nor the binding molecule is immobilized on a solid phase, and the like. Among the above, from the viewpoint of efficiency, for example, the contact mode in a state in which only either the protein (antigen) or the binding molecule is immobilized on a solid phase is preferable. In the contact mode in step (1a), when only either the protein (antigen) or the binding molecule is immobilized on a solid phase, the solid phase is preferably washed after immobilization.

The solid phase is not particularly restricted insofar as it is capable of immobilizing the target molecule of the present invention or the binding molecule to the target molecule of the present invention. Examples of the solid phase include plates, slides, films, and the like containing polystyrene, glass, nitrocellulose, and the like as the main component. The solid phase may be one coated with ingredients to more easily immobilize the target molecule of the present invention or the binding molecule to the target molecule of the present invention, such as readily reactive compounds (e.g., compounds having a readily reactive group, gold colloid, and the like). The compounds having a readily reactive group are those capable of forming a covalent bond with protein. Examples of the compounds include compounds having (1H-imidazol-1-yl)carbonyl groups, succinimidyloxycarbonyl groups, epoxy groups, aldehyde groups, amino groups, thiol groups, carboxyl groups, azide groups, cyano groups, active ester groups (1H-benzotriazol-1-yloxycarbonyl group, pentafluorophenyloxycarbonyl group, para-nitrophenyloxycarbonyl group, and the like), carbonyl halide groups (a carbonyl chloride group, a carbonyl fluoride group, a carbonyl bromide group, a carbonyl iodide group), and the like.

Examples of the compounds having readily reactive groups include epoxy silane, polylysine, and the like.

The solid phase is preferably subjected to blocking with a bovine serum albumin (BSA) buffer solution or the like.

The mode of measuring the amount or the concentration of the target molecule of the present invention in step (1b) is not particularly restricted, and an appropriate mode can be selected according to the type of the method for detecting the target molecule of the present invention described above (e.g., various kinds of immunoassays). The measurement can be performed by quantitatively determining a signal derived from the label of the used labeled substance, for example. As a more specific mode, the measurement can be performed by bringing the labeled antibody into contact with a complex of the target molecule of the present invention and the binding molecule to the target molecule of the present invention, and quantitatively determining a signal derived from the label of the bonded labeled antibody, for example.

One example of the mode includes ELISA. According to ELISA, the concentration of the target molecule of the present invention can be quantitatively determined by preparing a standard curve using a standard antibody. The standard antibody can be prepared from a commercially available antibody, such as by affinity purification using a biotin-labeled antigen and a streptavidin-agarose resin. First, wells in an appropriate ELISA plate are coated with antigens or antibodies, and then subjected to blocking with a BSA buffer solution. Next, a standard antibody or a sample at each concentration is added to each well and allowed to stand still for a specific time, followed by washing the wells, and then a secondary antibody labeled with peroxidase is added to each well and allowed to stand still for a specific time. Thereafter, the wells are washed, a peroxidase substrate is added to each well and allowed to stand still for a specific time to develop color, a reaction stopper, such as sulfuric acid, is added, and then the absorbance is measured using a plate reader. A standard curve is prepared based on the concentration of the standard antibody and the absorbance of the antibody, and then the concentration of the target molecule of the present invention in the sample is quantitatively determined based on the standard curve.

The amount of the target molecule of the present invention can be calculated based on the obtained signal amount. For example, in the case of non-competitive methods, the obtained signal amount can be used as it is as the amount of the target molecule of the present invention. As another example, in the case of competitive methods, the obtained signal amount is inversely proportional to the amount of the target molecule of the present invention. The amount of the target molecule of the present invention can be calculated from the obtained signal amount based on this relation.

The concentration of the target molecule of the present invention can be calculated by dividing the amount of the target molecule of the present invention by the amount of the biological sample or the component amount (e.g., the total amount of protein) in the biological sample.

The test method of the present invention, including step (1), can provide the amount and/or the concentration of a target biomarker serving as an index of the possibility of liver cancer development (e.g., stratification), which can assist in testing for the possibility of liver cancer development and the like.

In step (1), not only the detection or the measurement of the target molecule of the present invention but also the detection or the measurement of other biomarkers of the test for the possibility of liver cancer development can be performed. Examples of the other biomarkers include AFP, the FIB-4 index, and the like.

### 1-2. Step (2)

As one aspect, the test method of the present invention preferably further includes step (2) of determining the possibility of liver cancer development of the subject based on the amount or the concentration of the target molecule detected in step (1).

More specifically, step (2) can include:
step (2a) of determining that, when the amount or the concentration of the target molecule detected in step (1) is equal to or greater than the cutoff value, the possibility of liver cancer development of the subject is high; and/or
step (2b) of determining that, when the amount or the concentration of the target molecule detected in step (1) is equal to or smaller than the cutoff value, the possibility of liver cancer development of the subject is low.

The cutoff value is predetermined by preparing a database in which the amounts or the concentrations of the individual target molecules of the present invention and the development or non-development of liver cancer are tracked and by performing statistical analysis or ROC analysis of the data on the amounts or the concentrations of the target molecules of the present invention of those who develop liver cancer and those who do not develop liver cancer for an assessment group. Alternatively, the cutoff value can also be set every time. When the cutoff value is determined by statistical analysis, the median, the arithmetic mean, or other means of data on the amount or the concentration of the target molecule of the present invention of the assessment group can be used, for example. When the cutoff value is determined by ROC analysis, the cutoff value based on ROC analysis can be the amount or the concentration of the target molecule of the present invention at the point on the ROC curve at which the distance between the point 1.0 on the vertical axis (sensitivity or true positivity) of the ROC curve graph and the point 0.0 on the horizontal axis (1-specificity) is the smallest or can be the cutoff value derived from Youden's index (Cancer, 1950; 3:32-35.) of the ROC curve. After the database of the assessment group has been established, the database may be used without any change to set the cutoff value in a method for assessing the risk of liver cancer development of the subject of the present invention. Alternatively, the database may be used while the database of the assessment group of patients with chronic liver disease is being updated as appropriate by incorporating new patients with chronic liver disease, including the subject of the present invention, in the assessment group, to set the cutoff value in the method for assessing the risk of liver cancer development of the subject of the present invention. The cutoff value can be set to, for example, a percentile value of the value of the amount or the concentration of the target molecule of the present invention in a biological sample in a subject group serving as the reference, such as any value among 10th to 90th percentile values, any value among 30th to 70th percentile values, or any value among 40th to 60th percentile values.

For example, the cutoff value of the serum Fibulin-3 concentration can be, for example, 7 to 13 µg/mL, preferably 8 to 12 µg/mL, and more preferably 9 to 11 µg/mL in the case of a subject with non-alcoholic liver disease, and can be, for example, 11 to 21 µg/mL, preferably 12 to 20 µg/mL, and more preferably 13 to 19 µg/mL in the case of a subject with chronic hepatitis C.

For example, the cutoff value of the serum GPNMB concentration can be, for example, 12 to 18 µg/mL, preferably 13 to 17 µg/mL, and more preferably 14 to 16 µg/mL in the case of a subject with non-alcoholic liver disease, and can be, for example, 7 to 26 µg/mL, preferably 8 to 25 µg/mL, and more preferably 9 to 24 µg/mL in the case of a subject with chronic hepatitis C.

A high possibility of liver cancer development can be, for example, a possibility of liver cancer development within 5 years of 5% or more, 7% or more, 10% or more, 12% or more, 15% or more, 17% or more, or 20% or more, for example. The upper limit in this case is not particularly restricted and can be 50%, 40%, or 30%, for example.

A low possibility of liver cancer development can be, for example, a possibility of liver cancer development within 5 years of less than 5%, 4% or less, 3% or less, 2% or less, or 1% or less.

For a subject that has been determined to have a high possibility of liver cancer development, the frequency of a test for determining whether the subject suffers from liver cancer is preferably high (or increased frequency).

In this case, the frequency of the test can be, for example, once every 2 to 4 months.

For a subject that has been determined to have a low possibility of liver cancer development, the frequency of the test for determining whether the subject suffers from liver cancer with is preferably low (or reduced frequency).

In this case, the frequency of the test can be, for example, once every 12 to 60 months.

The test for determining whether a subject suffers from liver cancer includes, for example, blood tests, imaging tests, and the like.

### 2. Test drug for possibility of liver cancer development

In one aspect, the present invention relates to a test drug (in this specification, also described as "test drug of the present invention") for the possibility of liver cancer development containing the binding molecule to the target molecule of the present invention. A description of the test drug is given below.

The test drug of the present invention is a drug for testing the possibility of liver cancer development.

The test drug of the present invention may be in the form of a composition containing the binding molecule to the target molecule of the present invention. The composition may contain other ingredients as required. The other ingredients include, for example, bases, carriers, solvents, dispersants, emulsifiers, buffers, stabilizers, excipients, binders, disintegrants, lubricants, thickeners, moisturizers, coloring agents, flavors, chelating agents, and the like.

The test drug of the present invention may be in the form of a kit containing the binding molecule to the target molecule of the present invention. The kit may contain instruments, reagents, and the like that can be used in the implementation of the test method of the present invention.

The binding molecule to the target molecule of the present invention can also be in a state of being immobilized on any solid phase. Therefore, the test drug of the present invention can be provided in the form of a substrate on which the binding molecule to the target molecule of the present invention is immobilized (e.g., a microarray chip on which a probe is immobilized and the like; as other examples, an ELISA plate on which an antibody is immobilized and the like).

The solid phase used for the immobilization is not particularly restricted insofar as it is capable of immobilizing antibodies and the like, and includes, for example, glass plates, nylon membranes, microbeads, silicon chips, capillaries, and other substrates. The immobilization of a detection agent on the solid phase is not particularly restricted.

The instruments include, for example, test tubes, microtiter plates, agarose particles, latex particles, purification columns, epoxy-coated glass slides, gold colloid-coated glass slides, and the like.

The reagents include, for example, labeled antibodies, standard samples (positive controls and negative controls), and the like.

As the labeled antibodies, various commercially available labeled antibodies can be used according to the type (e.g., isotype) of the binding molecule to the target molecule of the present invention.

As the standard samples, the target molecule of the present invention is used. The target molecule of the present invention can be obtained by, for example, culturing a cell in which an expression vector of the target molecule of the present invention is introduced and purifying the cell or a culture supernatant.

### EXAMPLES

The present invention is described below in detail with reference to Examples, but the present invention is not limited to these Examples.

### Test Example 1. Test 1 of possibility of liver cancer development

The test was performed with the aim of clarifying usefulness of Fibulin as a predictive marker for hepatocarcinogenesis development in patients with chronic liver disease (patients with non-alcoholic fatty liver disease). 170 cases were targeted in which the patients were diagnosed as having non-alcoholic fatty liver disease by liver biopsy, and were followed up on so that their clinical courses were able to be understood. The diagnostic criteria followed a guideline. The blood of the patient was collected, followed by serum separation, and the serum was stored at -80°C for use. MBL Code No. CY-8120 CircuLex Human Fibulin-3/EFEMP1 ELISA Kit was used for the measurement. The serum was uniformly diluted 200-fold with a diluent included in the kit and measured. For the measurement, a Thermo Fisher Varioskan LUX was used.

Table 1 shows patient background. Of all 170 cases, 95 cases had non-alcoholic fatty liver (NAFL) and 75 cases had non-alcoholic steatohepatitis (NASH). In the histopathological diagnosis at the time of the liver biopsy, the diagnosis was made with Matteoni classification 1/2 as NAFL and 3/4 as NASH. Fibrosis was determined according to the NASH CRN scoring system of Kleiner et al. (criteria described in the guideline).

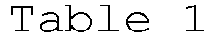

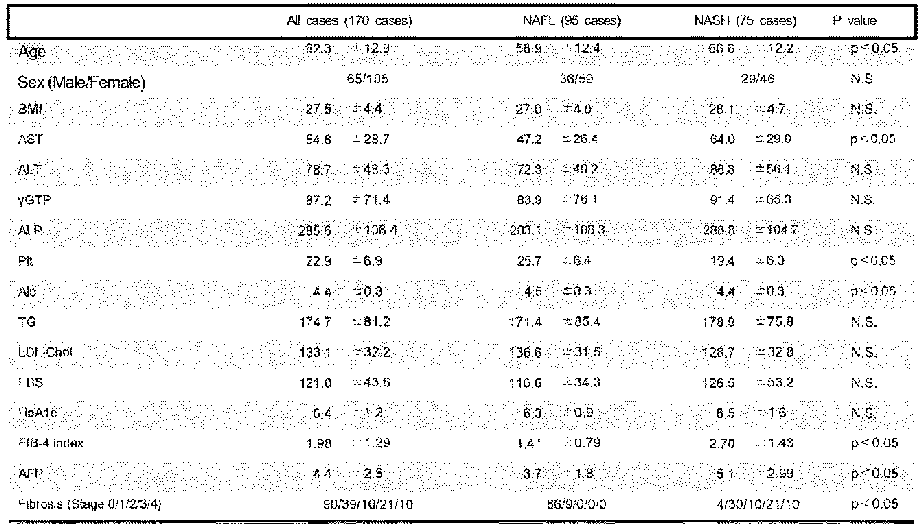

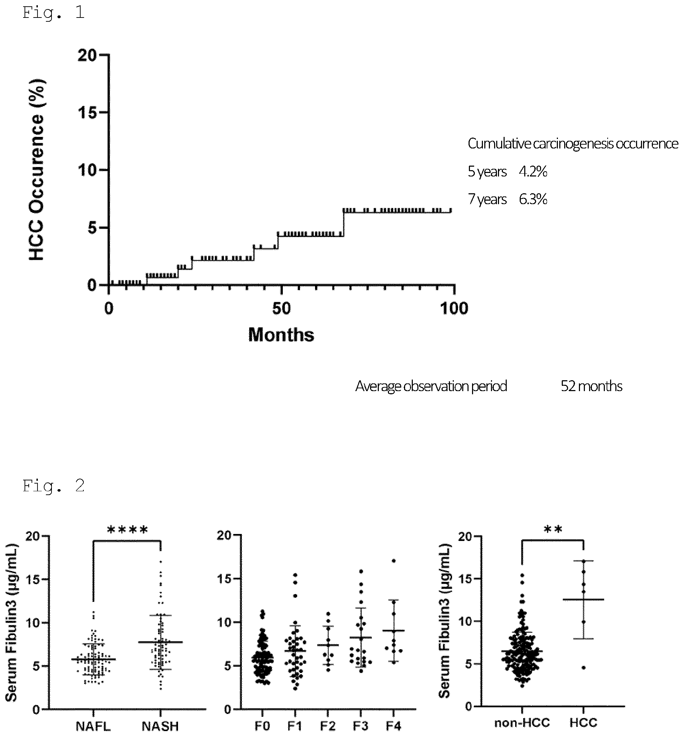

Fig. 1. illustrates the cumulative hepatocarcinogenesis occurrence of all patients with non-alcoholic fatty liver disease.

Fig. 2 illustrates the serum Fibulin-3 values of each patient group. It has been found that Fibulin-3 increased with the extension of fibrosis, and indicated a significantly high value in carcinogenesis cases.

For both the serum Fibulin-3 value and the FIB-4 index, the area under the ROC curve (AUROC) was calculated based on the time-dependent ROC curve. The threshold was set using Youden's index. Table 2 shows the results. It has been found that Fibulin-3 enables the prediction of hepatocarcinogenesis development.

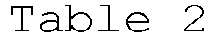

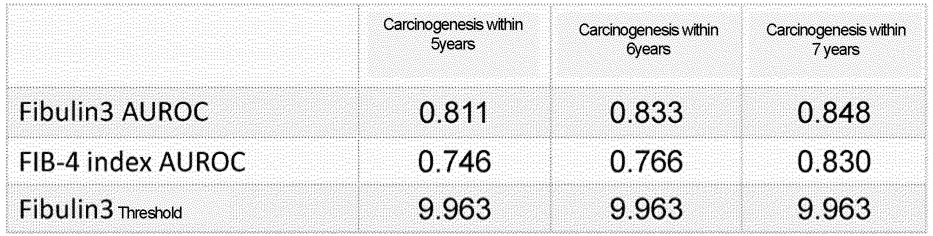

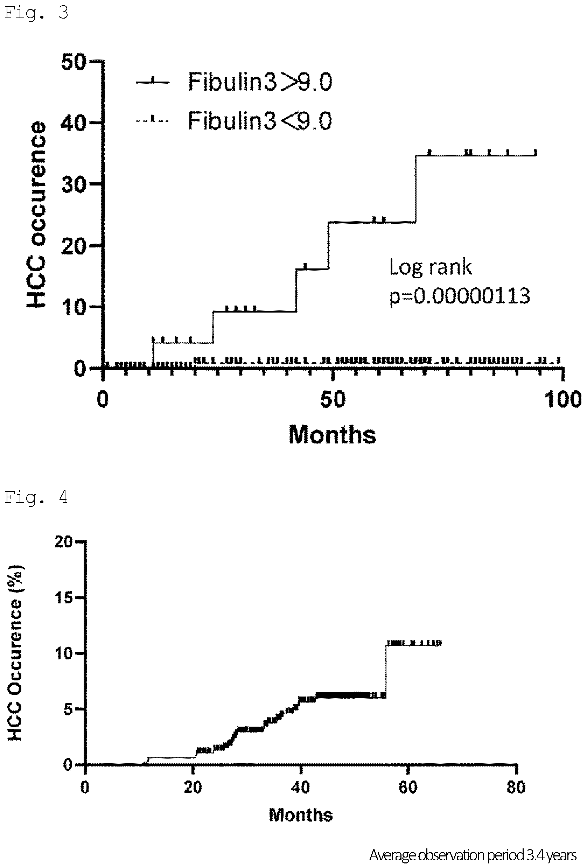

Fig. 3 illustrates the cumulative hepatocarcinogenesis occurrence of each of two groups classified according to the serum Fibulin-3 value (145 patients with less than 9.0 µg/mL and 25 patients with 9.0 µg/mL or more). As an assay method, the log-rank test was used. It has been found that Fibulin-3 enables stratification of liver cancer development.

### Test Example 2. Test 2 of possibility of liver cancer development

The test was performed with the aim of clarifying usefulness of Fibulin as a predictive marker for hepatocarcinogenesis development in patients with chronic liver disease (patients with chronic hepatitis C). 470 cases were targeted in which the patients underwent liver biopsy before DAA treatment for hepatitis C, achieved sustained viral response (SVR) with the DAA treatment, and had pre-treatment serum stored. The diagnosis and treatment of chronic hepatitis C were performed according to the hepatitis C guideline at that time. The serum Fibulin value was measured from the stored serum before treatment in the same manner as in Test Example 1, and the relation to hepatocarcinogenesis after SVR was examined. Although samples were usually diluted 200-fold, the samples that exceeded the standard range were further diluted three-fold for re-measurement, and the values were calculated.

Table 3 shows patient background.

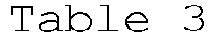

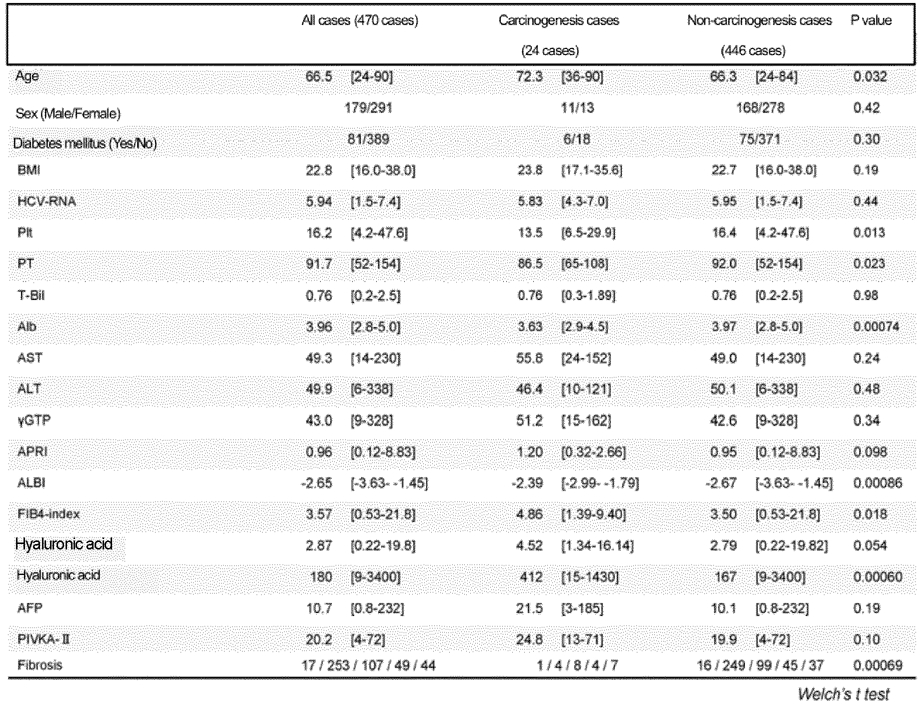

Fig. 4 illustrates the cumulative hepatocarcinogenesis occurrence after SVR in chronic hepatitis C.

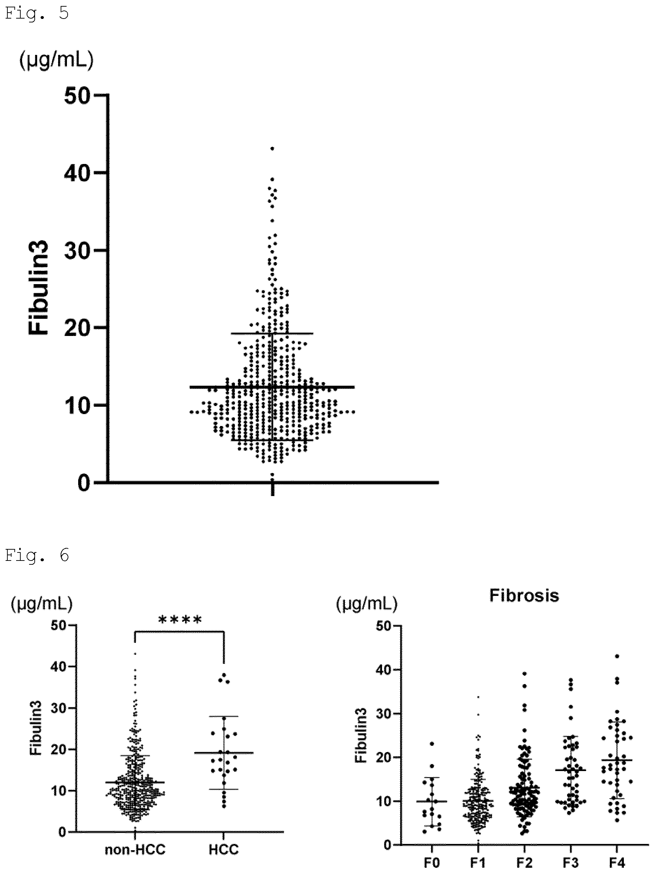

Fig. 5 illustrates the distribution of the serum Fibulin-3 values before treatment of chronic hepatitis C. The serum Fibulin-3 values before the treatment of hepatitis C were higher than the serum Fibulin-3 values in NAFLD.

Table 4 shows a patient background comparison based on the serum Fibulin-3 value.

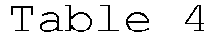

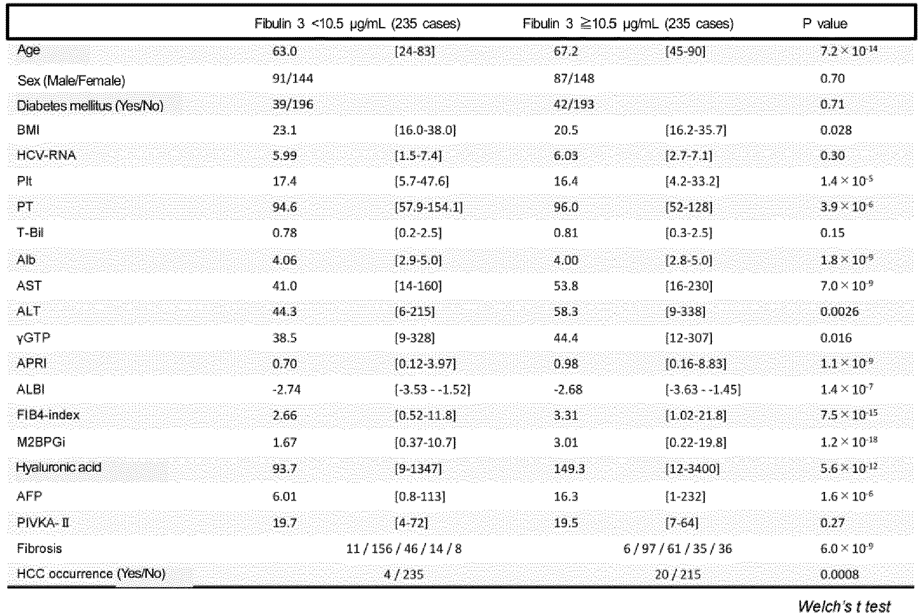

Fig. 6 illustrates the serum Fibulin-3 values of each patient group. It has been found that Fibulin-3 increased with the extension of fibrosis, and indicated a significantly high value in carcinogenesis cases.

Table 5 shows the results of univariate analysis and multivariate analysis. For the statistics, the Cox proportional hazards model was used. Fibulin-3 had the lowest univariate analysis p-value. The number of events was 24, and therefore multivariate analysis was performed using a model in which three items (Alb, hyaluronic acid, and Fibulin-3) were selected from those with the lowest p-values, and only Fibulin-3 was a significant factor.

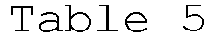

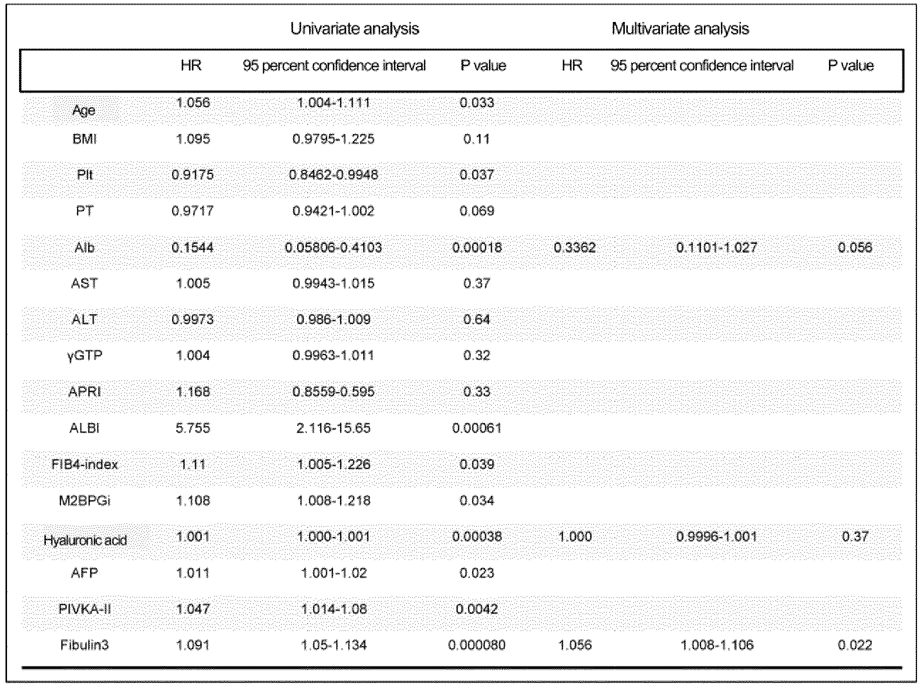

Table 6 shows the results of performing multivariate analysis in a model in which three factors (FIB-4index, AFP, and Fibulin-3) were selected, which had been reported in previous reports and the like. Also in this analysis, only Fibulin-3 was the significant factor.

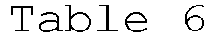

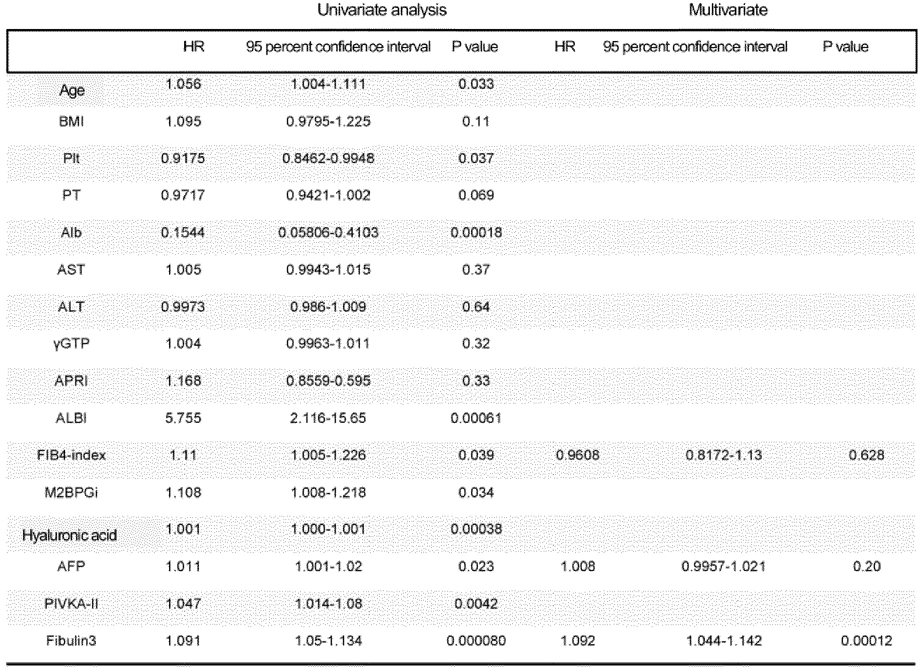

For both the serum Fibulin-3 value and the FIB-4 index, the AUROC was calculated based on the time-dependent ROC curve. The threshold was set using Youden's index. Table 7 shows the results. It has been found that Fibulin-3 enables the prediction of hepatocarcinogenesis development.

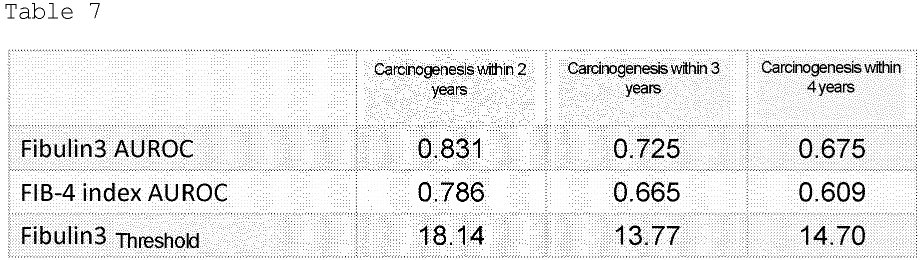

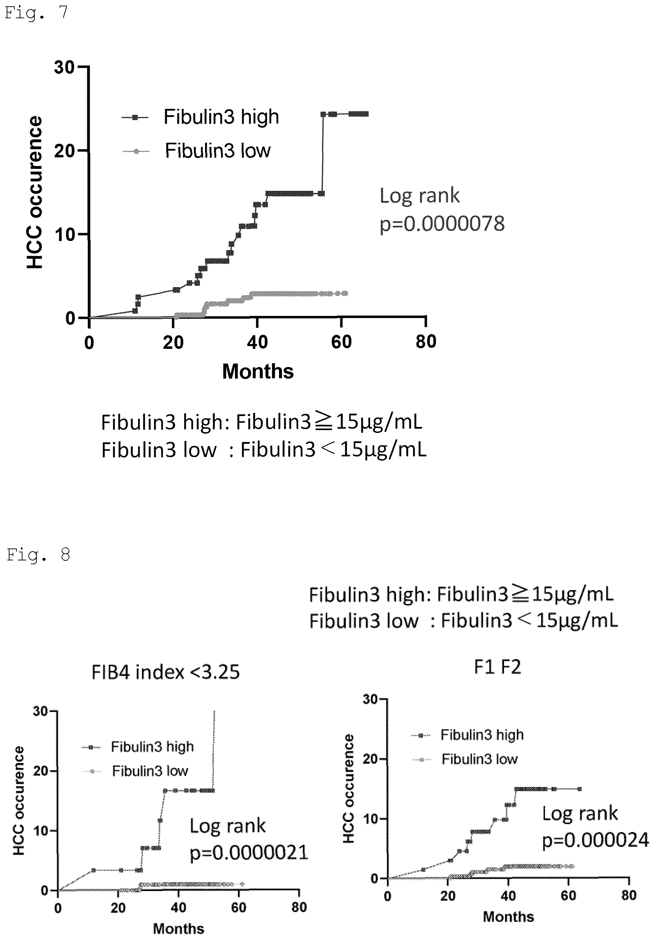

Fig. 7 illustrates the cumulative hepatocarcinogenesis occurrence of each of two groups classified according to the serum Fibulin-3 value (348 patients with less than 15.0 µg/mL and 122 patients with 15.0 µg/mL or more). As the assay method, the log-rank test was used. It has been found that Fibulin-3 enables stratification of liver cancer development.

Fig. 8 illustrates the cumulative hepatocarcinogenesis occurrence for each of two groups classified according to the serum Fibulin-3 value (348 patients with less than 15.0 µg/mL and 122 patients with 15.0 µg/mL or more) when the patient group was limited to a patient group with an FIB4 index of less than 3.25 or F1 and F2 patient groups.

### Test Example 3. Test 3 of possibility of liver cancer development

The test was performed with the aim of clarifying usefulness of GPNMB as a predictive marker for hepatocarcinogenesis development in patients with chronic liver disease (patients with non-alcoholic fatty liver disease). 170 cases were targeted in which the patients were diagnosed as having non-alcoholic fatty liver disease by liver biopsy, and were followed up on so that their clinical courses were able to be understood. The diagnostic criteria followed a guideline. The serum GPNMB value was measured from the stored serum at the time of the liver biopsy using Human Osteoactivin/GPNMB DuoSet ELISA (Catalog #DY2550 R&D SYSTEMS), and the relation to hepatocarcinogenesis was examined. The serum was diluted 20-fold with a diluent (1% BSA in PBS) for measurement. For the measurement, the numerical value of the absorbance of 450 nm to 540 nm was measured using a Thermo Fisher Varioskan LUX, and a calibration curve was prepared in a four-parameter logistic curve using a standard to measure the concentration of each sample.

Table 8 shows patient background. Of a total of 170 cases, 95 cases had non-alcoholic fatty liver (NAFL) and 75 cases had non-alcoholic steatohepatitis (NASH). In the histopathological diagnosis at the time of the liver biopsy, the diagnosis was made with Matteoni classification 1/2 as NAFL and 3/4 as NASH. Fibrosis was determined according to the NASH CRN scoring system of Kleiner et al. (criteria described in the guideline).

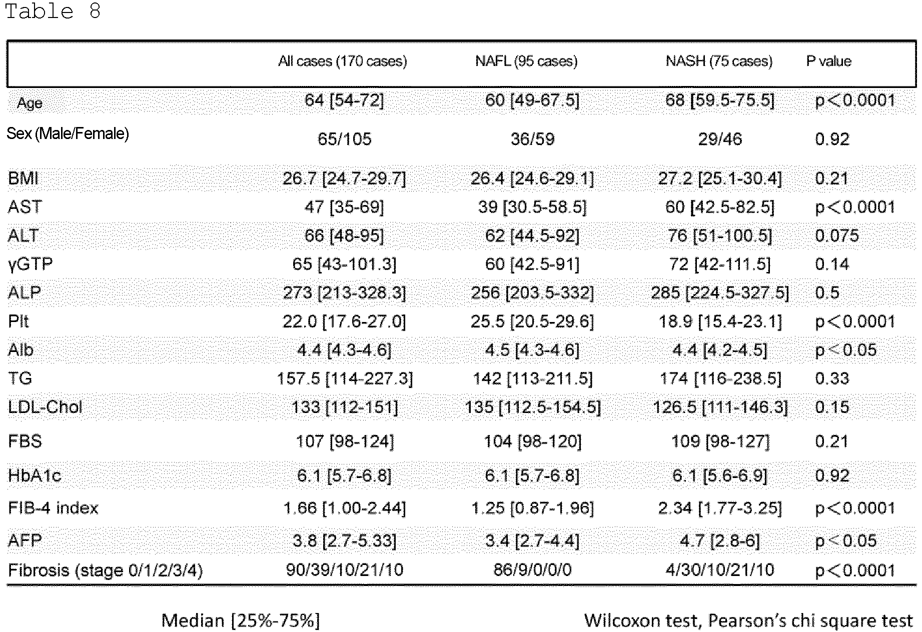

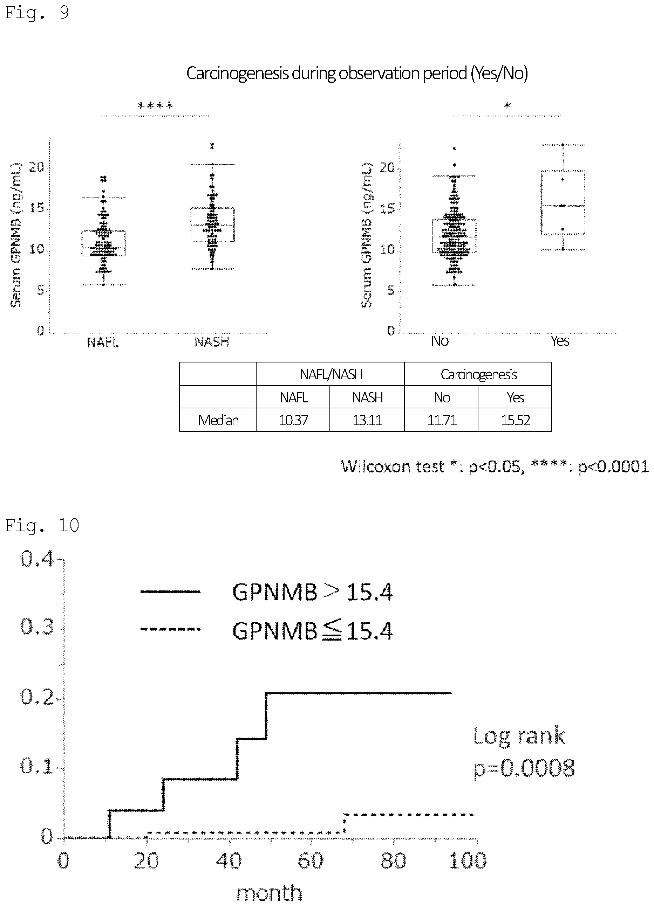

Fig. 9 illustrates the serum GPNMB values in each patient group. The serum GPNMB value increased in the NASH group as compared with the NAFL group and was significantly higher in carcinogenesis cases.

For both the serum GPNMB value and the FIB-4 index, AUROC was calculated based on the time-dependent ROC curve. The threshold was set using Youden's index. When carcinogenesis within 5 years was examined, the threshold for the serum GPNMB value was 15.468, the serum GPNMB value AUROC was 0.772, and the FIB-4 index AUROC was 0.746. Further, when carcinogenesis within 4 years was examined, the serum GPNMB value AUROC was 0.7113. When carcinogenesis within 5 years was examined, the serum GPNMB value AUROC was 0.7113.

Fig. 10 illustrates the cumulative hepatocarcinogenesis occurrence of each of two groups classified according to the serum GPNMB value (15.4 µg/mL or less, and more than 15.4 µg/mL). As an assay method, the log-rank test was used. It has been found that GPNMB enables stratification of liver cancer development.

### Test Example 4. Test 4 of possibility of liver cancer development

The test was performed with the aim of clarifying usefulness of GPNMB as a predictive marker for hepatocarcinogenesis development in patients with chronic liver disease (patients with chronic hepatitis C). 469 cases were targeted in which the patients underwent liver biopsy before DAA treatment for hepatitis C, achieved sustained viral response (SVR) with the DAA treatment, and had pre-treatment serum stored. The serum GPNMB value was measured from the stored serum before treatment in the same manner as in Test Example 3, and the relation to the hepatocarcinogenesis after SVR was examined.

Table 9 shows patient background.

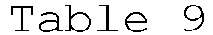

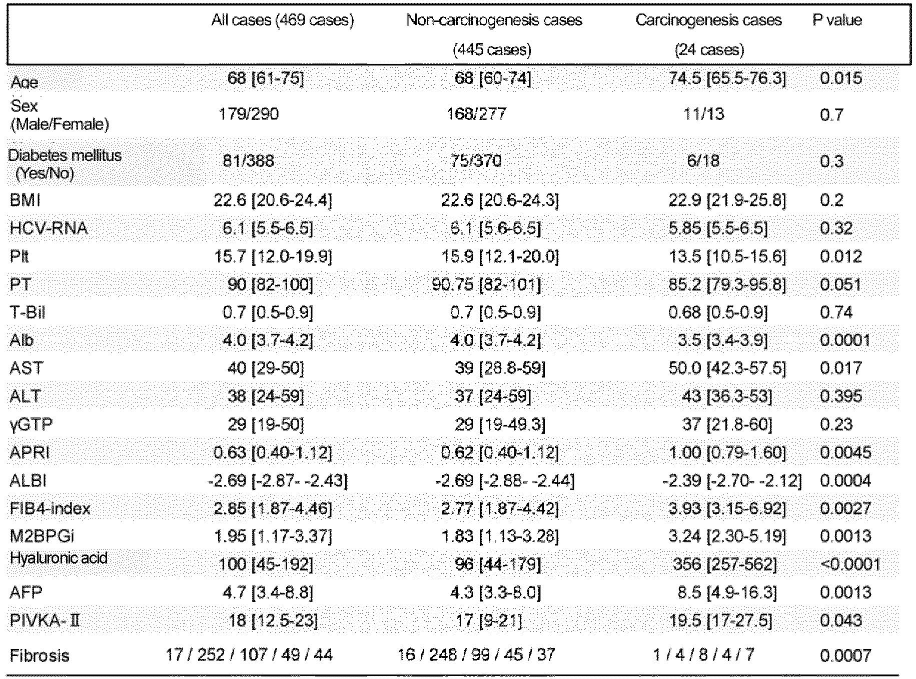

Table 10 shows the results of univariate analysis and multivariate analysis. For the statistics, the Cox proportional hazards model was used. GPNMB had the lowest univariate analysis p-value.

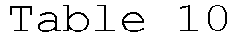

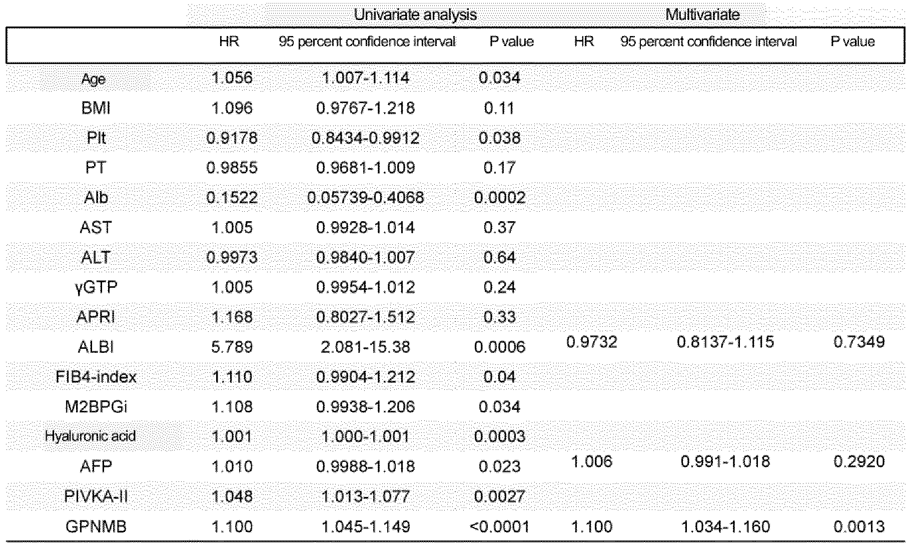

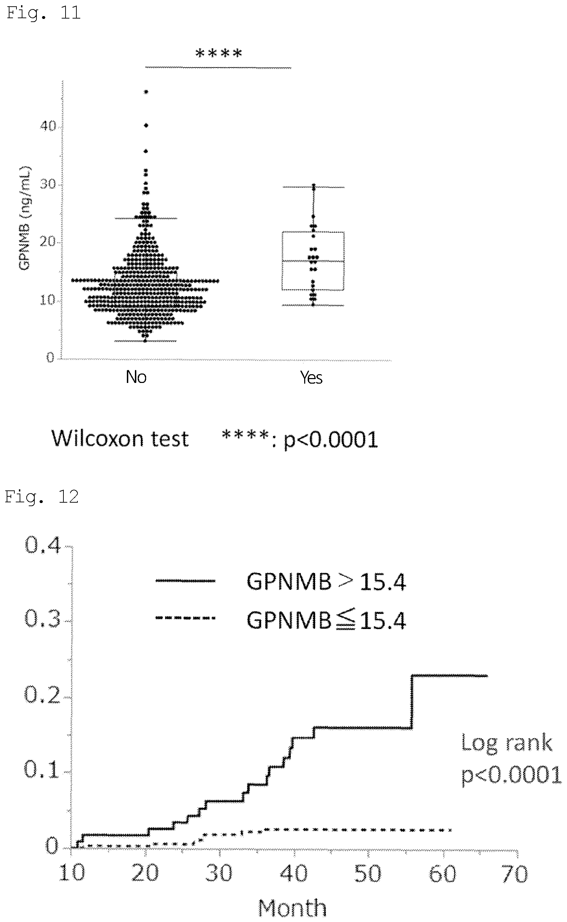

Fig. 11 illustrates the serum GPNMB value in the non-carcinogenesis group and the carcinogenesis group. It has been found that GPNMB indicates a significantly high value in the carcinogenesis cases.

For both the serum GPNMB value and the FIB-4 index, AUROC was calculated based on the time-dependent ROC curve. The threshold was set using Youden's Index. Table 11 shows the results. It has been found that GPNMB enables the prediction of hepatocarcinogenesis development.

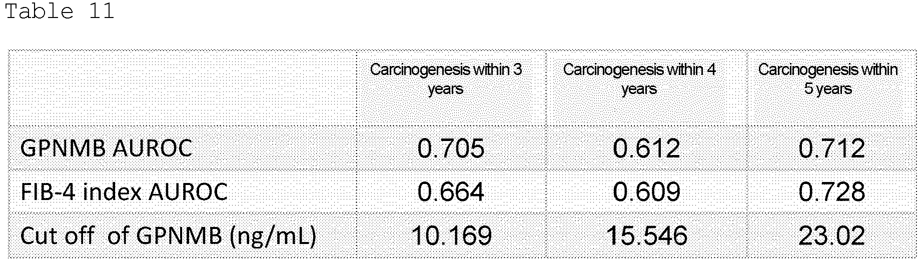

Fig. 12 illustrates the cumulative hepatocarcinogenesis occurrence of each of two groups classified according to the serum GPNMB value (15.4 µg/mL or less, and more than 15.4 µg/mL). As an assay method, the log-rank test was used. It has been found that GPNMB enables stratification of liver cancer development.

### Test Example 5. Test 5 of possibility of liver cancer development

The test was performed with the aim of clarifying usefulness of Fibulin as a predictive marker for hepatocarcinogenesis development in patients with chronic liver disease (patients with non-alcoholic fatty liver disease). 476 cases, including 170 cases used in Test Example 1, were targeted in which the patients were diagnosed as having non-alcoholic fatty liver disease by liver biopsy, and were followed up on so that their clinical courses were able to be understood. The diagnostic criteria followed a guideline. The blood of the patient was collected, followed by serum separation, and the serum was stored at -80°C for use. The MBL Code No. CY-8120 CircuLex Human Fibulin-3/EFEMP1 ELISA Kit was used for the measurement. The serum was uniformly diluted 200-fold with a diluent included in the kit and measured. For the measurement, a Thermo Fisher Varioskan LUX was used.

Table 12 shows the patient background. Stages 3 and 4 were defined as fibrosis extension cases according to the NASH CRN scoring system of Kleiner et al. (criteria described in the guideline).

**Table 12**

| | All (N=476) | Fibulin-3 low (N=238) | Fibulin-3 high (N=238) | p value |
|---|---|---|---|---|
| Age (years) | 61[ 17-87] | 52 [17-84] | 66 [17-87] | <0.001 |
| Sex (Male/Female) | 201/275 | 125/113 | 76/162 | <0.001 |
| BMI (kg/cm²) | 27.3 [16.8-53.3] | 27.7 [18.2-53.3] | 26.8 [16.8-48.9] | 0.21 |
| Diabetes mellitus (Yes/No) | 230/246 | 103/135 | 127/111 | 0.028 |
| Plt (x10³/µl) | 216 [56-637] | 226 [94-506] | 204 [56-637] | <0.001 |
| Alb (g/dl) | 4.4 [1.9-5.2] | 4.4 [1.9-5.2] | 4.3 [2.9-5.1] | 0.004 |
| T-Bil (mg/dl) | 0.7 [0.2-2.9] | 0.7 [0.2-2.3] | 0.7 [0.2-0.9] | 0.95 |
| AST (U/I) | 50 [15-300] | 45 [15-300] | 56 [19-245] | 0.03 |
| ALT (U/I) | 66 [12-501] | 71 [14-501] | 63 [12-275] | 0.04 |
| γGTP (U/I) | 62 [12-1070] | 60 [12-1070] | 64 [14-431] | 0.9 |
| AFP (ng/ml) | 3.6 [0.7-93] | 3.3 [0.8-93] | 4.1 [0.7-41] | 0.06 |
| HbA1c (%) | 6.0 [4.2-15.7] | 5.8 [4.2-11.6] | 6.1 [4.2-15.7] | 0.03 |
| TG (mg/dl) | 145 [38-605] | 157 [38-605] | 138 [49-530] | 0.15 |
| LDL-Chol (mg/dl) | 121 [34-254] | 124 [34-254] | 120 [50-229] | 0.28 |
| Type 4 Collagen 7S (ng/ml) | 4.9 [1.8-329] | 4.5 [1.8-11.1] | 5.4 [2.6-329] | 0.074 |
| Hyaluronic acid (ng/ml) | 56 [7-3099] | 32 [7-538] | 81 [9-3099] | <0.001 |
| M2BPGi (COI) | 0.87 [0.15-5.63] | 0.79 [0.18-4.23] | 0.96 [0.15-5.63] | <0.001 |
| FIB-4 index | 1.77 [0.26-10.6] | 1.22 [0.26-6.41] | 2.18 [0.31-10.6] | <0.001 |
| Liver cancer occurrence (Yes/No) | 18/458 | 3/235 | 15/223 | 0.001 |
| Decompensation event (Yes/No) | 18/458 | 3/235 | 15/223 | 0.001 |
| Observation period (years) | 5.0 [0.04-17.4] | 4.9 [0.06-17.4] | 5.2 [0.04-16.8] | 0.089 |
| Serum Fibulin-3 (µg/ml) | 4.9 [1.3-17.1] | 3.6 [1.3-4.9] | 6.7 [4.9-17.1] | <0.001 |

Table 13 shows the results of univariate analysis and multivariate analysis. For the statistics, the Cox proportional hazards model was used. Fibulin-3 had the second lowest univariate analysis p-value after the FIB-4 index. The number of events was 18, and therefore multivariate analysis was performed using a model in which three items (FIB-4 index, Fibulin-3, and M2BPGi) were selected from those with the lowest p-values, and two items (FIB-4 index and Fibulin-3) were significant factors.

**Table 13**

| | Univariate analysis | | Multivariate analysis | |
|---|---|---|---|---|
| | HR (95% CI) | p value | HR (95% CI) | p value |
| Age (years) | 1.03 (0.99-1.07) | 0.18 | | |
| Sex (Male/Female) | 1.40 (0.55-3.52) | 0.48 | | |
| BMI (kg/cm²) | 1.02 (0.91-1.13) | 0.77 | | |
| Diabetes mellitus (Yes/No) | 1.31(0.52-3.31) | 0.57 | | |
| Pit (x10³/µl) | 0.81 (0.73-0.88) | 4.2x10⁻⁶ | | |
| Alb (g/dl) | 0.22 (0.07-0.66) | 7.2x10⁻³ | | |
| T-Bil (mg/dl) | 0.99 (0.27-3.67) | 0.99 | | |
| AST (U/I) | 1.01 (1.00-1.02) | 0.070 | | |
| ALT (U/I) | 1.00 (0.99-1.01) | 0.65 | | |
| γGTP (U/l) | 1.00 (1.00-1.01) | 0.27 | | |
| AFP (ng/ml) | 1.03 (1.00-1.06) | 0.086 | | |
| HbA1c (%) | 0.76 (0.47-1.23) | 0.27 | | |
| TG (mg/dl) | 1.00 (0.99-1.00) | 0.20 | | |
| LDL-Chol (mg/dl) | 0.98 (0.96-1.00) | 0.010 | | |
| Type 4 Collagen 7S (ng/ml) | 1.01 (0.99-1.03) | 0.40 | | |
| Hyaluronic acid (ng/ml) | 1.00 (1.00-1.00) | 3.7x10⁻³ | | |
| M2BPGi (COI) | 1.90 (1.32-2.73) | 6.1x10⁻⁴ | 1.43 (0.82-2.49) | 0.21 |
| FIB-4 index | 1.66 (1.38-2.01) | 1.1x10⁻⁷ | 1.52 (1.18-1.47) | 1.3x10⁻³ |
| Serum Fibulin-3 (µg/ml) | 1.33 (1.19-1.49) | 8.3x10⁻⁷ | 1.26 (1.09-1.47) | 2.4x10⁻³ |

For both the serum Fibulin-3 value and the FIB-4 index, AUROC was calculated based on the time-dependent ROC curve. Table 13 shows the results. In this cohort, it also has been found that Fibulin-3 enables the prediction of hepatocarcinogenesis development.

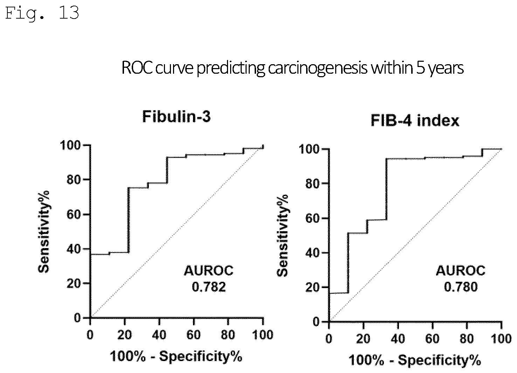

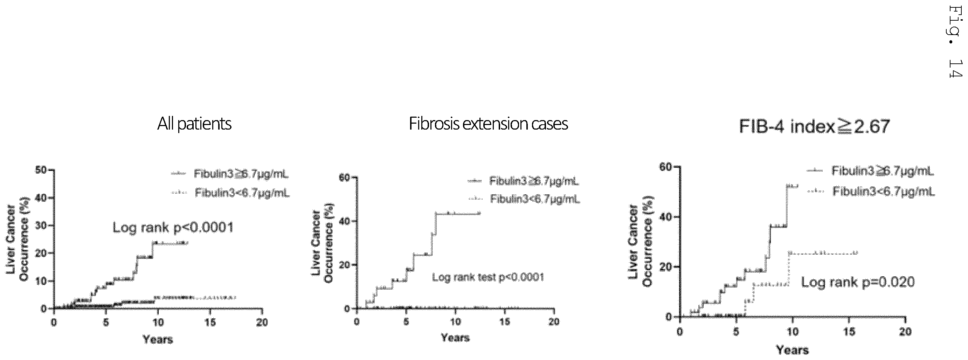

It was examined whether liver cancer development can be stratified using the threshold (6.7 µg/mL) calculated using Youden's index in the ROC curve illustrated in Fig. 13. Fig. 14 illustrates the cumulative hepatocarcinogenesis occurrence of each of two groups (6.7 µg/mL or more, and less than 6.7 µg/mL) classified according the Fibulin-3 value in the cases of a total of 476 patients, 120 patients with fibrosis extension, and 120 patients with a FIB-4 index of 2.67 or more in this order. As an assay method, the log-rank test was used. It has been found that Fibulin-3 enables the stratification of liver cancer development in all of the groups: all patients, fibrosis extension, and a FIB-4 index of 2.67 or more.

## Claims

1. A method for testing a possibility of liver cancer development comprising:
step (1) of detecting at least one target molecule selected from the group consisting of Fibulin-3, GPNMB, Fibulin-3 mRNA, and GPNMB mRNA in a biological sample collected from a subject.

2. The method according to claim 1, wherein step (1) includes:
step (1a) of bringing a binding molecule to the target molecule and the biological sample into contact with each other; and
step (1b) of measuring the amount or the concentration of the target molecule bonded to the binding molecule.

3. The method according to claim 1, further comprising:
step (2) of determining the possibility of liver cancer development of the subject based on the amount or the concentration of the target molecule detected in step (1).

4. The method according to claim 3, wherein step (2) includes:
step (2a) of determining that, when the amount or the concentration of the target molecule detected in step (1) is equal to or greater than a cutoff value, the possibility of liver cancer development of the subject is high; and/or
step (2b) of determining that, when the amount or the concentration of the target molecule detected in step (1) is equal to or smaller than the cutoff value, the possibility of liver cancer development of the subject is low.

5. The method according to any one of claims 1 to 4, wherein the subject is a subject with chronic liver disease.

6. The method according to any one of claims 1 to 4, wherein the target molecule is at least one member selected from the group consisting of Fibulin-3 and GPNMB.

7. The method according to any one of claims 1 to 4, wherein the biological sample is a body fluid or a sample derived from a body fluid.

8. The method according to claim 7, wherein the body fluid is at least one member selected from the group consisting of whole blood, serum, and plasma.

9. The method according to any one of claims 1 to 4, wherein the subject is a human.

10. A test drug for a possibility of liver cancer development comprising:
a binding molecule to at least one target molecule selected from the group consisting of Fibulin-3, GPNMB, Fibulin-3 mRNA, and GPNMB mRNA.

11. Use of a binding molecule to at least one target molecule selected from the group consisting of Fibulin-3, GPNMB, Fibulin-3 mRNA, and GPNMB mRNA for producing a test drug for a possibility of liver cancer development.

12. Use of a binding molecule to at least one target molecule selected from the group consisting of Fibulin-3, GPNMB, Fibulin-3 mRNA, and GPNMB mRNA as a test drug for a possibility of liver cancer development.

13. A binding molecule to at least one target molecule selected from the group consisting of Fibulin-3, GPNMB, Fibulin-3 mRNA, and GPNMB mRNA for use in testing a possibility of liver cancer development.
